# EUROPÄISCHE PATENTSCHRIFT

(11) **EP 4 300 140 B1**
(45) Veröffentlichungstag und Bekanntmachung des Hinweises auf die Patenterteilung: **11.03.2026**
(21) Anmeldenummer: 22182306.5
(22) Anmeldetag: 30.06.2022
(51) Int. Cl.: G01T 1/24

(54) **DETEKTORMODUL FÜR EINEN RÖNTGENDETEKTOR MIT EINER HEIZSCHICHT**
DETECTOR MODULE FOR AN X-RAY DETECTOR WITH A HEATING LAYER
MODULE DE DÉTECTEUR POUR UN DÉTECTEUR À RAYONS X DOTÉ D'UNE COUCHE CHAUFFANTE

(43) Veröffentlichungstag der Anmeldung: 03.01.2024
(73) Patentinhaber: Siemens Healthineers AG, 91301 Forchheim (DE)
(72) Erfinder: Hosemann, Michael, 91056 Erlangen (DE)
(74) Vertreter: Siemens Healthineers Patent Attorneys

(56) Entgegenhaltungen:
- US-A1- 2019 383 954
- US-B2- 10 866 328
- US-B2- 9 547 091

## Beschreibung

Die Erfindung betrifft ein Detektormodul für einen Röntgendetektor mit einer Heizschicht. Weiterhin betrifft die Erfindung einen eine Mehrzahl von Detektormodulen umfassenden Röntgendetektor sowie einen Computertomographie-Gerät umfassend einen solchen Röntgendetektor.

Ein Röntgendetektor wird in bildgebenden Anwendungen eingesetzt. So wird ein solcher Röntgendetektor beispielsweise für Computertomographie-Aufnahmen in der medizinischen Bildgebung genutzt, um ein räumliches Bild eines Untersuchungsbereiches eines Patienten zu erzeugen.

Röntgendetektoren können als zählende, direkt-konvertierende Röntgendetektoren oder integrierende, indirekt-konvertierende Röntgendetektoren ausgebildet sein. Ein Röntgendetektor, dessen Sensorschicht als eine direkt-konvertierende Halbleiterschicht ausgebildet ist, ermöglicht hierbei eine quantitative und energieselektive Erfassung einzelner Röntgenquanten. Zur Detektion der Röntgenquanten eignen sich beispielsweise Halbleitermaterialen in Form von CdTe, CdZnTe, CdTeSe, CdZnTeSe, CdMnTe, GaAs, Si oder Ge, die einen hohen Absorptionsquerschnitt für Röntgenstrahlung aufweisen. Beim Einfall von Röntgenstrahlung werden in der Sensorschicht Elektron-Loch-Paare, also Paare aus negativen und positiven Ladungsträgern, erzeugt. Durch eine an die Sensorschicht bzw. an die Oberfläche der Sensorschicht angelegte Spannung werden die Ladungsträger getrennt und bewegen sich zu den jeweils entgegengesetzt geladenen Elektroden bzw. Oberflächen der Sensorschicht. Der hierdurch verursachte Strom oder eine entsprechende Ladungsverschiebung kann von einer nachgeschalteten Sensorelektronik, im Folgenden auch Auswerteeinheit bzw. Auswerteschicht genannt, ausgewertet werden. Die Sensorschicht bzw. Sensoreinheit eines direkt-konvertierenden Röntgendetektors liegt dabei in der Regel in einem Stapelaufbau mit der ihr zugeordneten Auswerteeinheit vor. Solch ein Stapelaufbau aus Sensorschicht und Auswerteeinheit kann im Folgenden auch als Sensorboard bezeichnet werden.

Insbesondere direkt-konvertierende (photonenzählende) Röntgendetektoren müssen während des Betriebes möglichst exakt auf gleichbleibender Temperatur gehalten werden. Ansonsten können Bildartefakte auftreten. Bei direkt-wandelnden Röntgendetektoren bzw. bei den Detektormodulen eines entsprechenden Aufbaus ändert sich der elektrische Widerstand des Sensormaterials mit dem Röntgenfluss. Dies führt zu einer Änderung der Verlustleistung. Damit bedingt aber eine Änderung des Röntgenflusses eine Temperaturänderung in der Sensorschicht, wodurch wiederum die Energieauflösung und die Zählrate des Röntgendetektors beeinflusst werden. Zusätzlich zu einer zeitlich bedingten Temperaturänderung in der Sensorschicht wird das Driftverhalten eines Röntgendetektors auch von lokal unterschiedlichen Temperaturen der Sensorschicht beeinflusst. Derartige Temperaturgradienten entstehen insbesondere durch eine ungleichmäßige Entwärmung der Sensorschicht. Auch kann sich ein unerwünschter Temperaturgradient auf dem Sensorboard bzw. in der Sensorschicht des entsprechenden Sensorboards auch je nach Arbeitspunkt des Sensorboards ergeben. In Abhängigkeit der jeweils gewählten Einstellung der Betriebsparameter, wie beispielsweise die mittlere Betriebstemperatur des Detektormoduls bzw. des Sensormaterials oder die angelegte Versorgungsspannung, kann es zu einem erhöhten Strom durch das Sensormaterial kommen, der auch ohne einfallende Röntgenstrahlung zu einer hohen Verlustleistung und somit ebenfalls zu einem Temperaturgradienten in der Sensorschicht führen kann.

Zur Vermeidung von Temperaturgradienten in der Sensorschicht ist eine möglichst vollflächige thermische Ankopplung an einen Kühlkörper wünschenswert. Allerdings wird eine vollflächige Ankopplung und damit gleichmäßige Entwärmung beispielsweise durch an der Unterseite des Sensorboards angeordnete Bauteile, z.B. der Anbindung an eine Modulelektronik dienende Bauteile wie passive Bauelemente oder Stecker zur Datenübertragung und/oder durch sonstige mechanische Aussparungen, erschwert. Folglich ist die Entwärmung durch einen Kühlkörper stark von der Ausbildung des Sensorboards und der Geometrie des Modulaufbaus abhängig. Zur Stabilisierung der Temperatur des Röntgendetektors können weiterhin Heizelemente im Sensorboard verwendet werden, die zusätzliche Heizleistung einbringen können. Die Anordnung und Ausbildung der Heizelemente ist hier jedoch auf eine vorliegende, ggf. ungleichmäßige Temperaturverteilung im Sensorboard abzustimmen.

Für die Entwicklung eines Röntgendetektors ist es weiterhin wünschenswert einzelne Komponenten möglichst unabhängig voneinander zu entwickeln bzw. sich nicht zu früh im Designprozess festlegen und einschränken zu müssen. Weiterhin ist es vorteilhaft, wenn Komponenten flexibel beispielsweise über verschiedene Detektorgeometrien hinweg eingesetzt werden können. Dabei ist jedoch stets auch die Nutzung von kostenintensiven Bauteilen oder Herstellungsschritten auf ein sinnvolles Maß zu beschränken, was unter Umständen den zuvor genannten Zielen entgegenstehen kann.

US 2019/383954 A1 offenbart zum Beispiel einen Röntgendetektor mit einer Heizschicht.

Der Erfindung liegt als Aufgabe zugrunde, ein vorteilhaft temperaturstabilisierbares Detektormodul anzugeben, welches oben genannte Nachteile und Zielsetzungen zumindest zum Teil vorteilhaft berücksichtigt. Als weitere Aufgabe liegt der Erfindung zugrunde, einen Röntgendetektor sowie ein Computertomographiegerät mit einer Mehrzahl von entsprechenden Detektormodulen anzugeben.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Die Erfindung betrifft ein Detektormodul für einen Röntgendetektor umfassend in einem Stapelaufbau eine Sensorschicht, eine Ausleseschicht, eine Heizschicht, sowie eine der Heizschicht im Stapelaufbau nachgelagerte Verdrahtungseinheit, wobei die Heizschicht in eine Mehrzahl an Teilheizbereiche unterteilt ist, welche jeweils zumindest ein Heizelement aufweisen und jeweils einzeln für eine Leistungsversorgung kontaktierbar sind, und wobei mittels der nachgeordneten Verdrahtungseinheit die Teilheizbereiche der Heizschicht jeweils kontaktiert und zumindest eine Teilzahl der Teilheizbereiche für die Leistungsversorgung miteinander verschaltet sind.

Das erfindungsgemäße Röntgendetektormodul weist einen Stapelaufbau auf. Die Stapelrichtung kann insbesondere im Wesentlichen parallel zur betriebsgemäßen Einfallsrichtung der Röntgenstrahlung ausgerichtet sein. Die Sensorschicht ist dabei bevorzugt im Stapelaufbau der Röntgenstrahlungsquelle am nächsten angeordnet sein, so dass die Röntgenstrahlung insbesondere direkt auf die Konvertereinheit einfällt.

Zweckmäßigerweise umfasst die Sensorschicht ein direkt konvertierendes Halbleitermaterial, insbesondere Cadmiumtellurid (CdTe) oder Cadmiumzinktellurid (CdZnTe). Derartige Halbleiter-Materialien ermöglichen die Direktumwandlung der auf sie eintreffenden Röntgenstrahlung in ein elektrisches Signal und sind in guter Qualität in Bezug auf Ladungstransporteigenschaften und Homogenität kommerziell erhältlich. Die Sensorschicht kann auch ein anderes Halbleitermaterial in Form von CdTeSe, CdZnTeSe, CdMnTe, GaAs, Si oder Ge umfassen. Auch wenn die Temperaturstabilisierung in direkt-konvertierenden Detektoren eine besonders große Bedeutung innehat, kann eine erfindungsgemäße Ausbildung eines Detektormoduls auch eine Sensorschicht aufweisend ein indirekt-konvertierendes Material in Kombination mit einem Photodiodenarray umfassen. Als Konvertermaterial werden hier häufig Szintillatoren, beispielsweise GOS (Gd2O2S), CsJ, YGO oder LuTAG, eingesetzt.

Die Sensorschicht eines Detektormoduls kann einstückig ausgebildet sein. Sie kann jedoch auch aus mehreren aneinander gereihten Sensorelementen ausgebildet sein, welche zusammen die Sensorschicht aufspannen.

Die Ausleseschicht kann insbesondere jeweils eine Vielzahl an Pixelelektroniken umfassen, wobei eine jeweilige Pixelelektronik ausgebildet sein kann, zur pixelweisen und damit ortsaufgelösten Verarbeitung der von der Sensorschicht in eine Pixelelektronik eingespeisten elektrischen Signale zu einem digitalen Pixelmesssignal. Das heißt, eine jeweilige solche Pixelelektronik kann zumindest ausgebildet sein, ein von der Sensorschicht empfangenes elektrisches Signal weiterzuverarbeiten, insbesondere zu digitalisieren, beispielsweise mittels eines einen A/D-Wandlers (Analog-zu-Digital-Wandler). Die Pixelelektroniken können außerdem auch weitere Schaltelemente aufweisen, beispielsweise einen Signalverstärker oder einen Komparator. Dazu kann die Ausleseschicht, bzw. eine jeweilige Pixelelektronik der Ausleseschicht über elektrisch leitende Verbindungen mit der Sensorschicht gekoppelt sein. Die elektrisch leitenden Verbindungen zwischen einer Auswerteeinheit und einer Konvertereinheit können beispielsweise als Lotverbindungen, bspw. sogenannte Bumpbonds, als Leitklebeverbindungen oder auch anderweitig ausgebildet sein. Für möglichst geringe Einflüsse auf die Signalübertragung zwischen Sensorschicht und Ausleseschicht ist eine möglichst nahe Anordnung der Sensorschicht und der Ausleseschicht vorteilhaft.

Die Ausleseschicht kann in Form eines oder einer Mehrzahl an Auslesechips in Form eines integrierten Schaltkreises (IC, "integrated circuit") ausgebildet sein. Ein jeweiliger Auslesechip stellt dann jeweils Pixelelektroniken für die primäre Verarbeitung der elektrischen Signale von der Sensorschicht bereit. Insbesondere kann die Ausleseschicht einen oder auch mehrere anwendungsspezifische, integrierte Schaltkreise (ASIC(s), "application-specific integrated circuit") umfassen, welcher bzw. welche gemeinsam von der Ausleseschicht des Detektormoduls umfasst sind.

Die Ausleseschicht kann ein Gehäuse umfassen, in welchen ein solcher Auslesechip oder mehrere Auslesechips eingefasst ist bzw. sind. Weiterhin ist auch ein Verguss eines oder mehrere solcher Auslesechips als sogenannte ungehäuste "bare dies" (auch "bare chip", dt: nackter Chip) mit einer Vergussmasse, beispielsweise einem Gießharz, zu einem Bauteil möglich.

Die vom Stapelaufbau des Detektormoduls umfasste Heizschicht ist im Stapelaufbau thermisch mit der Sensorschicht gekoppelt. Sie deckt in einer zum Stapelaufbau senkrechten Projektionsebene die flächige Ausdehnung des Stapelaufbaus wenigstens teilweise ab. Eine Abdeckung durch die Heizschicht kann derart verstanden werden, dass über die der Heizschicht zugordneten Fläche eine Mehrzahl an Heizelemente vorgesehen sind, welche es ermöglichen, über diese Fläche der Heizschicht Heizleistung, d.h. Wärme, in den Stapelaufbau einzubringen. In bevorzugten Ausbildungen erstreckt sich die Heizschicht über zumindest den Großteil der flächigen Ausdehnung des Sensorschicht. Indem die Heizschicht einen Großteil dieser flächigen Ausdehnung abdeckt, kann vorteilhaft über den gesamten abgedeckten, flächigen Bereich eine Einbringung von Heizleistung über die Heizschicht und damit eine Temperaturstabilisierung ermöglicht sein. Je größer die dabei abgedeckte Fläche, desto vorteilhafter kann eine Einbringung von Heizleistung erfolgen. In besonders vorteilhaften Ausbildungen deckt die Heizschicht in einer zum Stapelaufbau senkrechten Projektionsebene die flächige Ausdehnung der Sensorschicht im Wesentlichen vollständig ab. Die Heizschicht kann insbesondere über die Ausleseschicht mit der Sensorschicht thermisch gekoppelt sein. In diesem Fall ist es vorteilhaft, wenn sich die Ausleseschicht, umfassend ein mögliches Gehäuse oder eine zumindest teilweise Ummantelung mittels einer Vergussmasse, in der zum Stapelaufbau senkrechten Projektionsebene über zumindest den Großteil der Fläche der Sensorschicht erstreckt. Derart kann ein Einbringen von Heizleistung in die Sensorschicht über die Ausleseschicht über eine möglichst große Fläche ermöglicht sein.

Die Heizschicht weist Heizelemente auf, welche für die Einbringung von Heizleistung in den Stapelaufbau ausgebildet sind, wenn diese im Betrieb mittels einer Leistungseinheit mit Leistung versorgt werden. Die Heizschicht erlaubt damit über die eingebrachte Heizleistung zu einer Temperaturstabilisierung des Detektormoduls beizutragen und beispielsweise Temperaturgradienten innerhalb des Stapelaufbaus bzw. der Sensorschicht entgegenzuwirken. Die Heizelemente können als einfache Heizwiderstände ausgebildet sein. Sie können als Leiterschleifen ausgebildet sein, welche von der Heizschicht umfasst sind. Diese können in ein nichtleitendes Material, insbesondere ein Plastikmaterial, eingebettet sein oder auf ein solches aufgebracht sein. Das nichtleitende Material kann beispielsweise einem Leiterplattenmaterial oder dem Gehäusematerial bzw. Vergussmaterial der Ausleseschicht entsprechen. Die Heizelemente der Heizschicht können im Wesentlichen innerhalb einer Ebene angeordnet sein.

Ein jeweiliger Teilheizbereich der Heizschicht deckt jeweils einen Teilbereich der flächigen Ausdehnung der Heizschicht ab. Das heißt, ein Teilheizbereich deckt einen Teilflächenbereich der von der Heizschicht abgedeckten Gesamtfläche des Stapelaufbaus ab. Jeder Teilheizbereich umfasst ein Heizelement, welches bei Versorgung mit Leistung ausgebildet ist, den jeweiligen vom Teilheizbereich abgedeckten Flächenbereich des Stapelaufbaus zu beheizen. Jeder Teilheizbereich ist individuell für eine Leistungsversorgung kontaktierbar. D.h. jeder Teilheizbereich weist Kontaktstellen auf, welche es ermöglichen das jeweilige Heizelement des Teilheizbereichs mit Leistung zu versorgen. Beispielsweise ist die Heizschicht in mehr als drei Teilheizbereiche unterteilt, konkret beispielsweise 9, 16, oder 25.

Die Unterteilung in Teilheizbereiche stellt eine durch die Heizschicht primär mögliche örtliche Auflösung bereit, in welcher eine in den Stapelaufbau einzubringende Heizleistung örtlich anpassbar ist, indem die Teilheizbereiche unterschiedlich angesteuert bzw. mit Leistung versorgt werden können. Je größer die Zahl an Teilheizbereichen, desto feiner kann die Heizleistungsverteilung mittels der Heizschicht und nachfolgenden Verdrahtungseinheit angepasst werden. Die Unterteilung kann gleichmäßig sein, d.h. die Teilheizbereiche können jeweils eine gleich große Teilfläche abdecken und regelmäßig über die Fläche der Heizschicht angeordnet sein. Insbesondere können die Teilheizbereiche der Heizschicht, gleichartig ausgebildet sein, d.h. ausgebildet sein bei gleichen Randbedingungen und gleicher Leistungsversorgung die gleiche Heizleistung abzugeben. Sie können insbesondere gleichartige Heizelemente umfassen. Sie können jedoch auch unterschiedliche Flächen abdecken und/oder unregelmäßig angeordnet sein. Eine im Wesentlichen gleichmäßige Anordnung oder gleichartige Ausbildung kann jedoch eine vorteilhaft einfache Umsetzung darstellen, da hier die Komplexität möglichst geringgehalten ist.

In dem erfindungsgemäßen Detektormodul ist der Heizschicht eine Verdrahtungseinheit nachgelagert, über welche die Teilheizbereiche für die Leistungsversorgung kontaktiert werden. Die Verdrahtungseinheit ist der Heizschicht in dem Sinne im Stapelaufbau nachgelagert, dass sie im Stapelaufbau entlang der betriebsgemäßen Strahleneinfallsrichtung nach der Heizschicht angeordnet ist. Die Verdrahtungseinheit folgt im Stapelaufbau direkt auf die Heizschicht.

Folgt die Verdrahtungseinheit im Stapelaufbau direkt auf die Heizschicht können die Kontaktstellen der Heizschicht und jeweilige auf der Verdrahtungseinheit vorgesehene Gegenkontaktstellen beispielsweise direkt mittels einer Lotverbindung kontaktiert werden. In anderen Ausführungen sind entsprechend geeignete Leitungen für die elektrisch leitende Verbindung zwischen der Heizschicht und der Verdrahtungsschicht vorgesehen. Weiterhin weist die Verdrahtungseinheit Leiterbahnen für die Zuführung der Leistung zu den Teilheizbereichen auf. Die Verdrahtungseinheit kann im Wesentlichen die gleiche flächige Ausdehnung aufweisen wie die Heizschicht. Zumindest weist die Verdrahtungseinheit eine derartige flächige Ausdehnung auf, welche es erlaubt die Kontaktstellen der Teilheizbereiche zu kontaktieren.

Die Verdrahtungseinheit ist weiterhin ein von der Heizschicht separates Bauteil des Detektormoduls, so dass eine unabhängige Herstellung und Anpassung der Verdrahtungseinheit ermöglicht ist, welche erst bei Zusammenbau des Detektormoduls mit der Heizschicht kontaktiert wird. Die Verdrahtungseinheit kann in einer bevorzugt kostengünstigen und einfachen Variante als Leiterplatte ausgebildet sein. Es ist jedoch auch beispielsweise eine Ausbildung in Form einer Trägerkeramik mit entsprechenden Leiterbahnen oder ähnliches denkbar.

Die Verdrahtungseinheit kann neben der Verdrahtung der Teilheizbereiche der Heizschicht außerdem auch als Trägereinheit für die Stapelanordnung und/oder als Substrat zur Signalübertragung von der Ausleseschicht des Detektormoduls zu einer nachgeordneten Modulelektronik oder auch andersherum dienen. Dazu können entsprechende Leitungen in der Verdrahtungseinheit vorgesehen sein, welche für die Signalübertragung ausgebildet sind.

Mittels der Verdrahtungseinheit wird zumindest eine Teilzahl der Teilheizbereiche für die Versorgung mit Leistung miteinander verschaltet. Eine Verschaltung umfasst, dass die Teilheizbereiche der Teilzahl über geeignete Leitungen zusammengeschaltet werden. Dies kann eine Parallelschaltung und/oder eine Reihenschaltung der Teilheizbereiche der Teilzahl umfassen. In der Regel liegen mehrere Teilzahlen vor, wobei die Teilheizbereiche einer jeweiligen Teilzahl miteinander verschaltet sind. Eine jeweilige Teilzahl kann dann für den Betrieb des Detektormoduls über die Verdrahtungseinheit an eine gemeinsame Leistungseinheit für die Versorgung mit Leistung angeschlossen sein. Für den Betrieb der Heizschicht können dafür weitere Leitungen vorgesehen sein, welche von der Verdrahtungseinheit zu einer bzw. mehreren Leistungseinheiten führen, welche jeweils ausgebildet sind, die an sie über die Verdrahtungseinheit angeschlossenen Teilheizbereiche mit Leistung zu versorgen.

Der Verschaltung der Teilheizbereiche liegt die Überlegung zugrunde, dass in der Regel Teilheizbereiche im Detektormodul vorliegen, welche im Betrieb des Detektormoduls einen ähnlichen oder gleichen Heizleistungsbedarf wie andere Teilheizbereiche des Detektormoduls aufweisen oder auch deren Heizleistungsbedarf in einem festen Verhältnis zueinander steht. Die geeignete Verschaltung dieser Teilheizbereiche mittels der Verdrahtungseinheit ermöglich es, diese Teilheizbereiche gemeinsam mit Leistung zu versorgen. Dies trägt zu einem besonders kosteneffizienten Einsatz des Detektormoduls bei, da für den Betrieb des Detektormoduls nicht für jeden einzelnen Teilheizbereich ein eigner Heizkreislauf mit einer eigenen Leistungseinheit vorgesehen sein muss, was einen hohen Aufwand für die Verkabelung bedingen und viele teure Leistungseinheiten zur Leistungsversorgung mit viel Platzbedarf erfordern würde. Die Umsetzung einer unterteilten Heizschicht und einer nachgeordneten Verdrahtungseinheit ermöglicht weiterhin eine möglichst generisch entworfene und universell einsetzbare Heizschicht im Detektormodul bereitzustellen, welche durch ihre bereitgestellte Unterteilung die Möglichkeit einer Anpassung an die konkret vorliegenden Bedingungen bereitstellt. Diese ist jedoch primär von weiteren Komponenten des Detektormoduls, etwa der Anordnung von Steckern zur Datenübertragung oder der Ausgestaltung eines anschließenden Kühlkörpers oder Modulträgers unabhängig und somit unabhängig von diesen entworfen und im Stapelaufbau umgesetzt werden kann. Eine Anpassung auf den konkret vorliegenden Heizleistungsbedarf des Detektormoduls für eine möglichst kosteneffektive Ansteuerung und Leistungsversorgung erfolgt dann durch die Verschaltung der Teilheizbereiche mittels der Verdrahtungseinheit. Mit einer derartigen Umsetzung kann das Detektormodul relativ einfach und kostensparsam auch noch zu einem späteren Zeitpunkt der Design- und Entwicklungsphase an konkrete, ggf. auch veränderte Bedingungen angepasst werden, indem lediglich die Verdrahtungseinheit des Detektormoduls angepasst werden muss. Weiterhin ist eine einfache Übertragbarkeit des Designs des Detektormoduls über verschiedene Detektortypen hinweg vereinfacht möglich. Beispielsweise kann derart eine möglichst generisch einsetzbare Einheit aus Sensorschicht, Ausleseschicht und Heizschicht für verschiedene Detektorgeometrien mit unterschiedlich ausgebildeten Kühlkörpern und Verkabelungen bereitgestellt werden, welche mittels der Verdrahtungseinheit dann an die tatsächlich vorliegende Verteilung des Heizbedarfs angepasst werden kann. Die Verdrahtungseinheit, beispielsweise in Form einer Leiterplatte, ist dabei günstig und einfach bereitstell- und anpassbar.

Die Verschaltung der Teilheizbereiche mittels der Verdrahtungseinheit ist in vorteilhaften Ausbildungen auf den Heizleistungsbedarf im Stapelaufbau, insbesondere der flächigen Verteilung des Heizleistungsbedarfs in einer zum Stapelaufbau senkrechten Projektionsebene, angepasst. Dieser kann beispielsweise im Betrieb eines vorläufigen Detektormoduls vermessen werden oder auch anhand von bekannten Geometrien und Anordnungen, beispielsweise der Ausbildung eines für das Detektormodul vorgesehenen Kühlkörpers, berechnet und/oder simuliert werden. Basierend auf solchen Überlegungen und Berechnungen kann eine vorzusehende Verschaltung abgeleitet und umgesetzt werden.

Die Verschaltung der Teilzahl der Teilheizbereiche mittels der Verdrahtungseinheit kann eine Parallelschaltung und/oder Reihenschaltung von Teilheizbereichen umfassen. Insbesondere können diejenigen Teilheizbereiche, welche Flächenbereichen des Stapelaufbaus mit gleichem Heizleistungsbedarf zugeordnet sind, derart miteinander verschaltet sein, dass sie an eine gemeinsame Leistungsversorgung anschließbar sind. Dies kann in zweckmäßigen Umsetzungen umfassen, dass diejenigen Teilheizbereiche der Heizschicht, welche Flächenbereichen des Stapelaufbaus mit gleichem Heizleistungsbedarf zugeordnet sind, in Parallelschaltung verschaltet sind. Diese können dann für eine gleichartige Leistungsversorgung und Ansteuerung an eine gemeinsame Leistungseinheit angeschlossen werden. Es kann jedoch auch eine Reihenschaltung von Teilheizbereichen vorgesehen sein.

In einer weiteren Ausbildung kann die Verschaltung der zumindest einen Teilzahl der Teilheizbereiche mittels der Verdrahtungseinheit eine Kombination aus einer Parallelschaltung und einer Reihenschaltung von Teilheizbereichen der Teilzahl umfassen, so dass durch die Verschaltung bei Versorgung mit Leistung ein festes Verhältnis zwischen den in den jeweiligen Teilheizbereichen vorliegenden Teilleistungen eingestellt ist. Derart können auch Teilheizbereiche, welche Flächenbereichen des Stapelaufbaus mit unterschiedlichem Heizleistungsbedarf zugeordnet sind an eine gemeinsame Leistungsversorgung angeschlossen werden, wobei je nach konkreter Verschaltung ggf. Bruchteile der durch die Leistungseinheit bereitgestellten Gesamtleistung als Teilleistung in den Teilbereichen zur Verfügung stehen können. Mittels einer geeigneten Kombination aus Parallelschaltung und Reihenschaltung von Teilheizbereichen mittels der Verdrahtungseinheit kann folglich ein relatives Verhältnis der jeweils in den verschalteten Teilheizbereichen erreichten Heizleistung festgelegt werden, wenn diese durch eine gemeinsame Leistungseinheit versorgt werden. Dies ist dann vorteilhaft, wenn durch ein derart festgelegtes Verhältnis dem Heizleistungsbedarf im Stapelaufbau auch Rechnung getragen werden kann. Bei günstigen geometrischen Verhältnissen kann derart die Anzahl an für den Betrieb des Detektormoduls notwendigen Leistungseinheiten und der Verkabelungsaufwand weiter reduziert werden. Insbesondere können mehrere Teilzahlen an Teilheizbereichen vorliegen, wobei zumindest eine dieser Teilzahlen mit einer Kombination aus einer Parallelschaltung und einer Reihenschaltung verschaltet sein kann. Andere Teilzahlen können auch lediglich mittels Parallelschaltungen oder Reihenschaltungen verschaltet sein.

Das Detektormodul kann insbesondere eine Anzahl an Leistungseinheiten umfassen, wobei jeweils eine über die Verdrahtungseinheit miteinander verschalteten Teilzahl an Teilheizbereiche an eine gemeinsame Leistungseinheit für die Versorgung mit Leistung angeschlossen ist. Die Anzahl der Leistungseinheiten richtet sich nach der Anzahl an miteinander verschalteten Teilzahlen an Teilheizbereichen. Die Leistungseinheit bzw. -einheiten können auf einer vom Stapelaufbau separaten Elektronikeinheit angeordnet sein, beispielsweise eine Leiterplatine, wobei vom Stapelaufbau zu den Leistungseinheiten geeignete Leitungen führen. Eine separate Elektronikeinheit kann vorteilhaft ausreichend Platz für die Anordnung der Leistungseinheiten bieten, wohingegen der Stapelaufbau selbst möglichst kompakt gehalten werden kann, was einer Aneinanderreihbarkeit der Stapelaufbauten für eine vergrößerte Detektionsfläche und ggf. auch deren optimalen Entwärmung entgegenkommen kann. Dabei ist der Austausch einzelner Komponenten bei einem Defekt ebenfalls erleichtert möglich.

In vorteilhaften Ausbildungen basiert die von einer jeweiligen Leistungseinheit bereitgestellte Leistung für die an sie angeschlossenen Teilheizbereiche auf einer erwarteten und/oder gemessenen Temperatur im Stapelaufbau. Derart kann vorteilhaft der Heizleistungsbedarf im Stapelaufbau und damit die notwendige Heizleistung abgestimmt werden und die jeweilige Leistungseinheit entsprechend angesteuert werden. Insbesondere kann vorteilhaft ein Temperaturverlauf während des Betriebs oder verschiedenen Betriebsparameter und/oder Umgebungsparameter des Detektormoduls berücksichtigt werden. Insbesondere kann dies umfassen, dass eine Temperaturverteilung in einer zum Stapelaufbau senkrechten Projektionsebene und ggf. auch deren zeitlicher Verlauf bzw. deren Abhängigkeit von verschiedenen Betriebs- und/oder Umgebungsparametern gemessen und/oder berechnet bzw. geschätzt wird, wobei die von einer jeweiligen Leistungseinheit bereitgestellte Leistung basierend auf der Temperaturverteilung eingestellt oder geregelt wird. Vorteilhaft wird die jeweils bereitgestellte Leistung einer Leistungseinheit auf den vorliegenden Heizleistungsbedarf der dieser Leistungseinheit zugeordneten Teilheizbereiche angepasst. Eine erwartete Temperatur bzw. Temperaturverteilung kann auf vorab durchgeführten Messungen im Betrieb des Detektormoduls basieren. Eine erwartete Temperatur bzw. Temperaturverteilung kann auf Berechnungen oder Simulationen basieren. Besonders bevorzugt basiert die Einstellung bzw. Regelung einer bereitgestellten Leistung durch eine jeweilige Leistungseinheit auf einer gemessenen Temperatur bzw. Temperaturverteilung im Stapelaufbau. Vorteilhaft kann eine direkte Temperaturinformation für eine Einstellung bzw. Regelung der Leistungseinheiten eingesetzt werden. Insbesondere kann eine jeweilige Leistungseinheit eine Regelschaltung aufweisen und ausgebildet sein, eine bereitgestellte Leistung basierend auf einer Temperatur im Stapelaufbau zu regeln.

Gemäß einer Ausführungsvariante ist dafür zumindest ein Temperatursensor im Stapelaufbau angeordnet. Derart kann eine Einstellung bzw. Regelung der Leistung vorteilhafterweise basierend auf zumindest einem gemessenen Temperaturwert im Stapelaufbau basieren und die aktuell gemessene Temperatur berücksichtigt werden. In vorteilhaften Ausbildungen sind mehrere Temperatursensoren im Stapelaufbau angeordnet, so dass eine aktuelle Temperaturverteilung in einer zum Stapelaufbau senkrechten Projektionsebene anhand von Messwerten verbessert ortsaufgelöst erfasst werden kann. Darauf basierend kann die Ansteuerung und/oder Regelung der in den Teilheizbereichen bereitgestellten Leistung umgesetzt sein. Es kann jedoch auch beispielsweise basierend auf einem einzelnen Temperaturmesswert eine erwartete Temperaturverteilung in einer zum Stapelaufbau senkrechten Projektionsebene abgeleitet werden. Eine derartige Umsetzung kann umfassen, dass vorab in Messungen und/oder Simulationen die zu erwartende Temperaturverteilung im Stapelaufbau im Betrieb des Detektormoduls in Verbindung mit einem gemessenen Temperaturmesswert vermessen wurde. Ausgehend von dem gemessenen Temperaturmesswert und der davon abgeleiteten erwarteten Temperaturverteilung kann dann die Ansteuerung und/oder Regelung der Heizleistung in den Teilheizbereichen umgesetzt sein. In einer vorteilhaft zweckmäßigen Ausführungsvariante ist der mindestens eine Temperatursensor in die Ausleseschicht integriert.

In vorteilhaften Ausgestaltungen des Detektormoduls ist die Heizschicht auf einer Oberfläche der Ausleseschicht aufgebracht oder in die Ausleseschicht integriert. Eine möglichst nahe Anordnung der Heizschicht relativ zur Sensorschicht ist vorteilhaft, da die Temperatur des Sensors der für die Bildqualität entscheidende Parameter ist. Die Anbringung auf einer Oberfläche der Ausleseschicht oder die Integration in die Ausleseschicht stellt weiterhin eine einfach bereitzustellende Ausführungsvariante dar, da hier notwendige Heizelemente und Leitungen einfach integrierbar und aufbringbar sind. Die Heizelemente der Heizschicht können beispielsweise in ein Gehäuse oder eine Ummantelung der Ausleseschaltkreise der Ausleseschicht eingebettet oder auf einer Oberfläche eines Gehäuses oder Ummantelung der Ausleseschaltkreise aufgebracht sein. Die Heizelemente der Heizschicht können in Form von Heizwiderständen bzw. Heizschleifen ausgebildet sein, welche auf einer Oberfläche der Ausleseschicht aufgebracht oder in die Ausleseschicht integriert sind. Für eine einfache Kontaktierbarkeit der Heizelemente der Heizschicht durch eine nachfolgende Verdrahtungseinheit kann es besonders zweckmäßig sein, die Heizschicht an bzw. auf einer Seite der Ausleseschicht vorzusehen, welche von der Sensorschicht abgewandt ist. Dies kann auch für eine möglichst ungestörte Übertragung der Signale von der Sensorschicht an die Auswerteschicht vorteilhaft sein.

Weiterhin kann der Stapelaufbau des erfinderischen Detektormoduls auf einem Modulträger angeordnet sein, welcher über den Stapelaufbau thermisch mit der Sensorschicht gekoppelt ist. Der Modulträger kann zur mechanischen Fixierung eines jeweiligen Detektormoduls in einem das Detektormodul umfassenden Geräts ausgebildet sein. Dazu können entsprechende Fixierungsmittel und Ausrichtungsmittel am Modulträger vorgesehen sein. Der Modulträger ist vorzugsweise als metallischer Kühlkörper ausgebildet, welcher für die Temperaturstabilisierung des Detektormoduls die Abfuhr von Wärme aus dem Stapelaufbau und insbesondere der Sensorschicht ermöglicht. Die Verdrahtung der Heizschicht ist dann insbesondere auf die Ausgestaltung des Modulträger und damit auf Bereiche unterschiedlich guter Wärmeabfuhr angepasst und bewirkt eine Vergleichmäßigung der Temperaturverteilung im Stapelaufbau.

Zweckmäßigerweise ist die Verdrahtungseinheit im Stapelaufbau über den Modulträger mit einer Modulelektronik verbunden. Die Modulelektronik kann für eine Auslese, Sammlung und/oder Weiterverarbeitung der Messdaten von der Ausleseschicht dienen. Die Modulelektronik kann hierzu weitere aktive oder passive Bauelemente aufweisen, welche ausgebildet sind, die benötigten Funktionalitäten bereitzustellen. Die Modulelektronik kann weiterhin der Übertragung von Ansteuerungsdaten und der Leistungsversorgung für den Betrieb des Stapelaufbaus dienen.

Insbesondere kann die Anzahl an Leistungseinheiten für die Versorgung der Heizschicht mit Leistung von der Modulelektronik umfasst sein. Die Modulelektronik bietet in der Regel ausreichend Platz für die Anordnung der Leistungseinheiten. Vorteilhaft sind die Leistungseinheiten trotzdem in relativer Nähe zu einem jeweiligen Stapelaufbau vorgesehen. Dabei ist der Austausch einzelner Komponenten bei einem Defekt ebenfalls erleichtert möglich.

Weiterhin kann das Detektormodul gemäß einer Ausführung eine Anzahl von einander benachbart auf dem Modulträger angeordneten Stapelaufbauten umfassen. Es können beispielsweise 2, 3 oder 4 identisch ausgebildeter Stapelaufbauten von dem Detektormodul umfasst sein. Vorteilhaft kann durch die Aneinanderreihung eine größere Ausdehnung der Gesamtdetektionsfläche des Moduls erreicht werden. Die Mehrzahl an Stapelaufbauten können einer gemeinsamen Modulelektronik zugeordnet sein und über diese versorgt oder angesteuert werden. Die Leistungsversorgung der Heizschicht eines jeden Stapelaufbaus ist bevorzugt unabhängig von den anderen steuer- oder regelbar.

Derart können ggf. unterschiedliche Randbedingungen der Stapelaufbauten, wie beispielweise eine einseitige Luftzufuhr bei der Kühlung des Detektormoduls, berücksichtig werden. Hierbei strömt eine zur Kühlung eingesetzte Kühlluft entlang des Detektormoduls und erwärmt sich. Entsprechend wird die Sensorschicht eines am Anfang der Kühlluftstrecke angeordneten Sensorboards eines Detektormoduls stärker gekühlt als die Sensorschicht eines am Ende der Kühlstrecke angeordneten Sensorboards.

Die Erfindung betrifft weiterhin einen Röntgendetektor zur Bildaufnahme eines von Röntgenstrahlung durchstrahlten Objektes, umfassend eine Mehrzahl von einander benachbart angeordneten Detektormodulen gemäß einer der zuvor beschriebenen Varianten. Die Anzahl der in einem Röntgendetektor eingesetzten Detektormodule und damit die Anzahl der Sensorboards sind abhängig von deren Größe sowie von der benötigten Sensorgesamtoberfläche. Der Röntgendetektor kann weitere Elemente umfassen, beispielweise Trägerkonstruktionen für die Anordnung der Detektormodule, Kühlluftzu- und -abführungen für eine verbesserte Wärmeabfuhr von den Detektormodulen oder ein Streustrahlengitter, welches in Strahleneinfallsrichtung vor der Sensorschicht der Detektormodule angeordnet ist.

Alle Ausgestaltungsvarianten, die zuvor in Zusammenhang mit dem erfindungsgemäßen Detektormodul beschrieben sind, können entsprechend auch im Röntgendetektor ausgeführt sein. Die im Hinblick auf das Detektormodul erfolgte Beschreibung und die zuvor beschriebenen Vorteile des Detektormoduls können entsprechend auch auf den erfindungsgemäßen Röntgendetektor übertragen werden.

Insbesondere betrifft die Erfindung weiterhin ein Computertomographiegerät umfassend einen Röntgendetektor wie zuvor beschrieben. Das Computertomographiegerät umfasst in Gegenüberstellung dazu eine Röntgenquelle, welche ausgebildet ist, den Röntgendetektor mit Röntgenstrahlung zu belichten. Röntgenquelle und Röntgendetektor sind dabei auf einem Rotor angeordnet, welcher eine Rotation um eine Drehachse ermöglicht. Das Computertomographiegerät kann dabei auch mehr als einen Röntgendetektor umfassen.

Für die Aufnahme eines Computertomographie-Bilddatensatzes wird in der Regel entlang der Rotationsachse zwischen die Röntgenquelle und die Detektoreinheit ein abzubildendes Objekt platziert und mittels der Röntgenquelle durchstrahlt.

Im Rahmen der Erfindung können außerdem Merkmale, welche in Bezug auf unterschiedliche Ausführungsformen der Erfindung beschrieben sind, zu weiteren Ausführungsformen der Erfindung kombiniert werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, der durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist das Computertomographiegerät das Computertomographiegerät auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:
Fig. 1 eine beispielhafte Ausbildung eines Detektormoduls für einen Röntgendetektor mit einer Mehrzahl an nebeneinander angeordneten Stapelaufbauten,
Fig. 2 ein Stapelaufbau eines beispielhaften Detektormoduls,
Fig. 3 eine schematische Veranschaulichung einer Heizschicht mit einer Mehrzahl an Teilheizbereichen eines beispielhaften Detektormoduls,
Fig. 4 eine Aufsicht auf einen beispielhaften Modulträger,
Fig. 5 eine schematische Veranschaulichung einer räumlichen Verteilung des Heizleistungsbedarfs in einem Stapelaufbau,
Fig. 6 eine schematische Veranschaulichung der Verschaltung der Mehrzahl an Teilheizbereichen eines beispielhaften Detektormoduls gemäß einer ersten Variante,
Fig. 7 eine schematische Veranschaulichung der Verschaltung der Mehrzahl an Teilheizbereichen eines beispielhaften Detektormoduls gemäß einer zweiten Variante, und
Fig. 8 ein Computertomographiegerät.

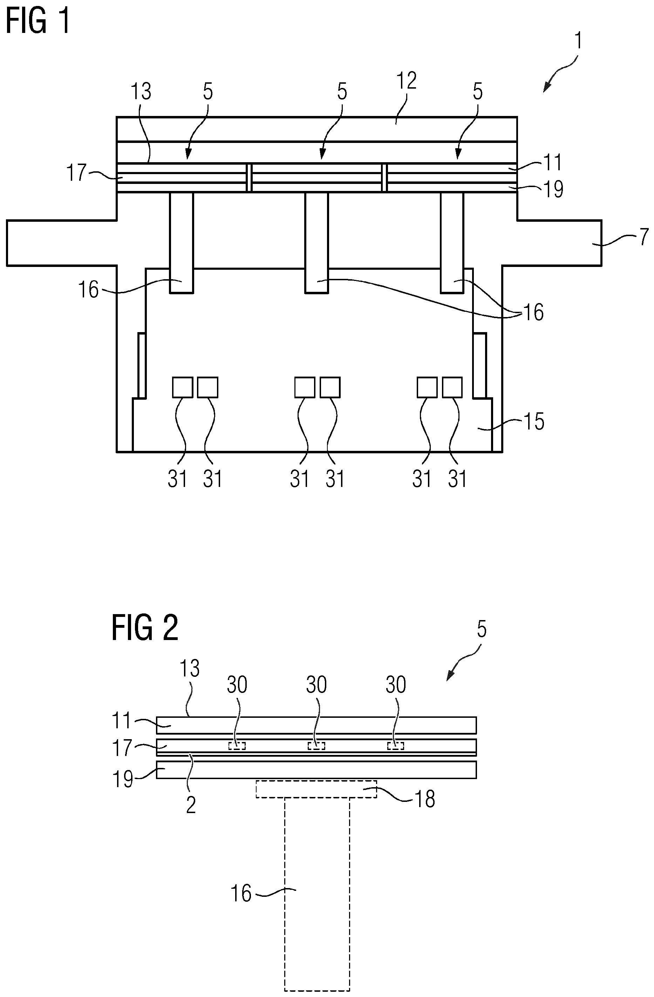

Figur 1 zeigt eine beispielhafte Ausbildungsvariante eines Detektormoduls 1 für einen Röntgendetektor. Die gezeigte Ausführung umfasst eine Mehrzahl an identisch ausgebildeter, nebeneinander angeordneter Stapelaufbauten 5. Ein erfindungsgemäßen Detektormodul kann jedoch auch anderweitig ausgebildet sein. Beispielsweise kann ein erfindungsgemäßes Detektormodul 1 auch nur einen Stapelaufbau 5 umfassen. Ein Stapelaufbau 5 umfassend eine Sensorschicht 11, eine Auswerteschicht 17, eine Heizschicht 2 (hier der Übersicht halber nicht explizit gezeigt) und eine Verdrahtungseinheit 19, wie er in einem erfindungsgemäßen Detektormodul 1 zum Einsatz kommen kann, wird im Folgenden anhand der Figur 2 näher erläutert werden.

Im Falle des in Fig. 1 gezeigten beispielhaften Detektormoduls 1 sind die Stapelaufbauten 5 auf einem metallische Modulträger 7 angeordnet. Diese sind weiterhin über den Modulträger 7 mit einer Modulelektronik 15 verbunden, von der ausgehende Datenleitungen 16 zu den jeweiligen Stapelaufbauten 5, Sensorboards genannt, verlaufen. Die Modulelektronik 15 kann für eine Auslese, Sammlung und/oder Weiterverarbeitung der Messdaten von der Ausleseschicht 19 dienen. Die Modulelektronik 15 kann weiterhin der Übertragung von Ansteuerungsdaten und der Leistungsversorgung für den Betrieb des Stapelaufbaus 5 dienen. Insbesondere kann eine Anzahl an Leistungseinheiten 31 für die Versorgung der erfindungsgemäßen Heizschicht 2 eines jeweiligen Stapelaufbaus 5 mit Leistung von der Modulelektronik 15 umfasst sein bzw. auf ihr angeordnet sein. Die Modulelektronik 15 bietet in der Regel ausreichend Platz für die Anordnung der Leistungseinheiten 31. Weiter wird die Sensoroberfläche 13 eines jeweiligen Stapelaufbaus von einem Kollimator 12 überdeckt, der zur Erzeugung eines parallelen Strahlenverlaufs und der Vermeidung von Streustrahlung dient.

Der Modulträger 7 dient hier weiterhin als Kühlkörper zur Entwärmung der Stapelaufbauten 5. Der Modulträger 7 ist über den Stapelaufbau 5 thermisch mit der Sensorschicht 11 eines jeweiligen Stapelaufbaus 5 gekoppelt.

Ein Ausführungsvariante eines Stapelaufbau 5 eines erfindungsgemäßen Detektormoduls 1, wie er auch von einem Modul wie in Fig. 1 umfasst sein kann, ist in Fig. 2 genauer veranschaulicht. Der Stapelaufbau 5 umfasst die Sensorschicht 11 mit der Sensoroberfläche 13. Die Sensorschicht 11 dient der Detektion von Röntgenstrahlung. Hierzu wird im eingebauten Zustand innerhalb eines Röntgendetektors über eine nicht gezeigte Elektrode eine Hochspannung an die Sensoroberfläche 13 angelegt. Die Sensorschicht umfasst in bevorzugten Ausbildungen ein direkt konvertierendes Halbleitermaterial, insbesondere Cadmiumtellurid (CdTe) oder Cadmiumzinktellurid (CdZnTe).

Der Stapelaufbau 5 umfasst weiterhin eine Ausleseschicht 17. In dem gezeigten Detektormodul 1 ist die Sensorschicht 11 der Ausleseschicht 17 aufgebracht. Zwischen der Ausleseschicht 17 und der Sensorschicht 11 sind, hier nicht gezeigte, elektrisch leitende Verbindungen, beispielsweise Lotverbindungen in Form von sogenannten "bump bonds", vorgesehen, welche eine Signalübertragung zwischen der Sensorschicht 11 und der Ausleseschicht 17 ermöglichen.

Die Ausleseschicht 17 kann beispielsweise einen oder auch mehrere anwendungsspezifische, integrierte Schaltkreise (ASIC(s), "application-specific integrated circuit") umfassen, welcher bzw. welche von einem Gehäuse eingefasst ist. In alternativen Varianten ist auch ein Verguss eines oder mehrere solcher Auslesechips als sogenannte ungehäuste "bare dies" (auch "bare chip", dt: nackter Chip) mit einer Vergussmasse, beispielsweise einem Gießharz, zu einem Bauteil möglich.

Der Stapelaufbau 5 umfasst weiterhin eine Heizschicht 2, sowie eine der Heizschicht 2 im Stapelaufbau nachgelagerte Verdrahtungseinheit 19. In dem gezeigten Beispiel ist die Heizschicht 2 auf eine Oberfläche der Ausleseschicht 17 aufgebracht. Die Heizschicht 2 kann auf einem Gehäuse oder einer Ummantelung der Ausleseschicht 17 mit einer Vergussmasse aufgebracht sein. In alternativen Varianten kann die Heizschicht jedoch auch in die Ausleseschicht 17, beispielsweise in ein Gehäuse, integriert sein. Die Heizschicht 2 weist Heizelemente 27 in Form von Heizwiderständen bzw. Leitschleifen auf, welche für die Einbringung von Heizleistung in den Stapelaufbau 5 ausgebildet sind, wenn diese im Betrieb mittels einer Leistungseinheit 31 mit Leistung versorgt werden. Die Heizwiderstände können beispielsweise direkt auf das Gehäuse oder die Ummantelung der Ausleseschicht 17 aufgebracht sein oder, alternativ, schichtartig in dieses bzw. diese eingebettet sein.

Die Heizschicht 2 ist in dem gezeigte Ausführungsbeispiel im Stapelaufbau 5 über die Ausleseschicht 17 thermisch mit der Sensorschicht 11 gekoppelt. Sie deckt gemäß einer vorteilhaften Ausbildung in einer zum Stapelaufbau 5 senkrechten Projektionsebene die flächige Ausdehnung des Stapelaufbaus 5 und damit auch die der Sensorschicht 11 vollflächig ab. Eine Erstreckung der Heizschicht 2 über zumindest den Großteil der flächigen Ausdehnung des Sensorschicht 11 ist vorteilhaft, da somit über den gesamten abgedeckten, flächigen Bereich eine Einbringung von Heizleistung über die Heizschicht 2 und damit eine Temperaturstabilisierung ermöglicht ist. Es kann jedoch auch Ausführungen geben, wobei keine vollflächige Abdeckung erreicht ist.

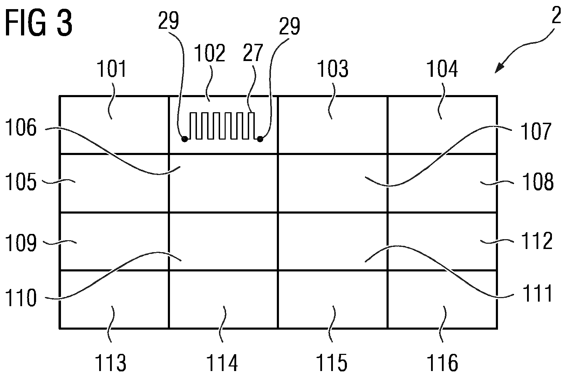

Die Heizschicht 2 ist, wie in Fig. 3 exemplarisch angedeutet, in eine Mehrzahl an Teilheizbereiche 101,102,...116 unterteilt, welche jeweils zumindest ein Heizelement 27 aufweisen (hier nur in Teilheizbereich 102 dargestellt) und jeweils einzeln für eine Versorgung mit Leistung kontaktierbar sind. Dafür weist jedes Heizelement 27 eines Teilheizbereichs 101,102,...116 Kontakte 29 auf, worüber das jeweilige Heizelement 27 des Teilheizbereichs 101,102,...116 mit Leistung versorgt werden kann.

Ein jeweiliger Teilheizbereich 101,102,...116 der Heizschicht 2 deckt einen Teilbereich der flächigen Ausdehnung der Heizschicht 2 in Fig. 2 ab. Das heißt, ein Teilheizbereich deckt einen Teilflächenbereich der von der Heizschicht 2 abgedeckten Gesamtfläche des Stapelaufbaus 5 ab und ist ausgebildet diesen zu beheizen, wenn das Heizelement 27 mit Leistung versorgt. In der gezeigten Veranschaulichung sind die Teilheizbereiche 101,102,...116 gleichmäßig und gleichartig ausgebildet. Es kann jedoch auch andere Ausführungen geben. Beispielsweise können unterschiedlich große Flächen von einem jeweiligen Teilheizbereich abgedeckt sein. Insbesondere können sich auch die Heizelemente 27 voneinander unterscheiden.

Mittels der Verdrahtungseinheit 19 werden die Teilheizbereiche 101,102,...116 der Heizschicht 2 für die Leistungsversorgung jeweils kontaktiert und zumindest eine Teilzahl der Teilheizbereiche 101,102,...116 für die Versorgung mit Leistung miteinander verschaltet. Eine Verschaltung umfasst, dass die Teilheizbereiche der Teilzahl über geeignete Leitungen zusammengeschaltet werden. Eine jeweilige Teilzahl kann dann für den Betrieb des Detektormoduls 1 über die Verdrahtungseinheit 19 an eine gemeinsame Leistungseinheit 31 für die Versorgung mit Leistung angeschlossen sein. Dazu weist die Verdrahtungseinheit 19 Leiterbahnen für die Zuführung der Leistung zu den Teilheizbereichen 101,102,...116 auf.

Die Verschaltung kann eine Parallelschaltung oder eine Reihenschaltung der Teilheizbereiche der Teilzahl umfassen. Insbesondere können diejenigen Teilheizbereiche, welche Flächenbereichen des Stapelaufbaus 5 mit gleichem Heizleistungsbedarf zugeordnet sind, derart miteinander verschaltet sein, dass sie an eine gemeinsame Leistungseinheit 31 anschließbar sind und gemeinsam mit Leistung versorgt werden. Eine Verschaltung der zumindest einen Teilzahl der Teilheizbereiche mittels der Verdrahtungseinheit 19 kann auch eine Kombination aus einer Parallelschaltung und einer Reihenschaltung von Teilheizbereichen der Teilzahl umfassen, so dass durch die Verschaltung bei Versorgung mit Leistung ein festes Verhältnis zwischen den in den jeweiligen Teilheizbereichen vorliegenden Teilleistungen eingestellt ist. In der Regel liegen mehrere Teilzahlen vor, wobei die Teilheizbereiche einer jeweiligen Teilzahl miteinander verschaltet sind und gemeinsam mit Leistung versorgt werden können.

Die Verdrahtungseinheit 19 im beispielhaften Stapelaufbau 5 in Fig. 2 folgt direkt auf die Heizschicht 2, so dass die Kontaktstellen 29 der Heizschicht und jeweilige auf der Verdrahtungseinheit 19 vorgesehene Gegenkontaktstellen vorteilhaft einfach direkt beispielsweise mittels einer Lotverbindung kontaktiert werden können. Es sind jedoch auch andere Ausführungen denkbar.

Die Verdrahtungseinheit 19 kann neben der Verdrahtung der Teilheizbereiche 101,102,...116 der Heizschicht 2 außerdem auch als Trägereinheit für die Stapelanordnung und/oder als Sub-Pastrat zur Signalübertragung von der Ausleseschicht 17 des Detektormoduls zu einer nachgeordneten Modulelektronik oder auch andersherum dienen. Dazu können entsprechende Leitungen in der Verdrahtungseinheit 19 vorgesehen sein, welche für die Signalübertragung ausgebildet sind.

Über einen Stecker 18 und Leitungen 16 kann die Verdrahtungseinheit 18 weiterhin mit nachfolgenden Komponenten verbunden sein. So können darüber für den Betrieb der Heizschicht 2 Leitungen vorgesehen sein, welche von der Verdrahtungseinheit 19 zu einer, ggf. zu einer Mehrzahl an Leistungseinheiten 31 führen, welche ausgebildet sind, an sie über die Verdrahtungseinheit 19 angeschlossenen Teilheizbereiche 101,102,...116 mit Leistung zu versorgen.

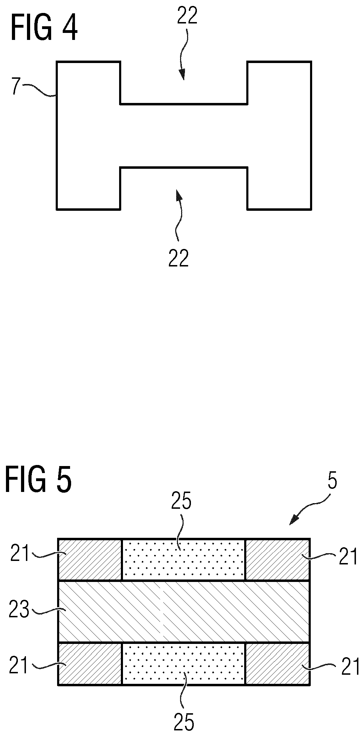

Anhand von Fig. 4 und 5 soll eine mögliche Abhängigkeit des Heizleistungsbedarfs im Stapelaufbau 5 eines Detektormoduls 1 von einem mechanischen Design eines Modulträgers 7 für das Detektormodul 1 veranschaulicht werden. Fig. 4 zeigt eine Aufsicht auf die geometrische Ausgestaltung eines Modulträger 7, wie er für einen einzelnen Stapelaufbau 5 vorgesehen sein kann. Der Modulträger in dieser Ausbildung weist Aussparungen 22 für eine Steckverbindung 18 zum Stapelaufbau 5 und/oder für Datenleitungen 16 von den Stapelaufbauten 5 zu einer nachgeordneten Modulelektronik 15 auf. Dies hat zur Folge, dass der Stapelaufbau 5 nicht vollflächig auf dem Modulträger 7 aufliegt, was zu einer ungleichmäßigen Entwärmung des Stapelaufbaus 5 durch den Modulträger 7 führt.

Fig. 5 zeigt eine mögliche daraus resultierende räumliche Verteilung des Heizleistungsbedarfs in der zum Stapelaufbau 5 senkrechten Projektionsebene, wenn dieser auf einem solchen Modulträger 7 platziert ist. In den Bereichen 25, welche zu den Bereichen der Aussparungen 22 korrespondieren, findet nur eine geringe Wärmeabfuhr statt. Dadurch ist der Heizbedarf gering. Über den Mittelsteg des Modulträgers wird viel Wärme abgeführt, demzufolge ist der Heizbedarf im Bereich 23 hoch.

An den seitlichen Stützen bzw. den Bereichen 21 kann der Heizbedarf beispielsweise zwischen den beiden Extremen liegen.

Eine erfindungsgemäße generische Heizschicht 2, wie sie beispielsweise in Fig. 2 schematisch veranschaulicht ist, kann mittels einer geeigneten Verschaltung mittels der Verdrahtungseinheit 19 vorteilhaft auf eine derartige erwartete Verteilung des Heizleistungsbedarfs abgestimmt werden um eine kosteneffiziente Bereitstellung und Betrieb des Detektormoduls 1 zu gewährleisten. In gleicher Weise könnte auch ein Stapelaufbau 5 mit einer generischen Heizschicht 2 in relativ einfacher Weise, und zwar lediglich durch Anpassen der Verdrahtungseinheit 19, auf andere mechanische Designs des Modulträgers 7 angepasst werden, wobei gleichzeitig eine möglichst nahe und vorteilhafte Anordnung der Heizschicht 2 relativ zur Sensorschicht 11 gewährleistet werden kann.

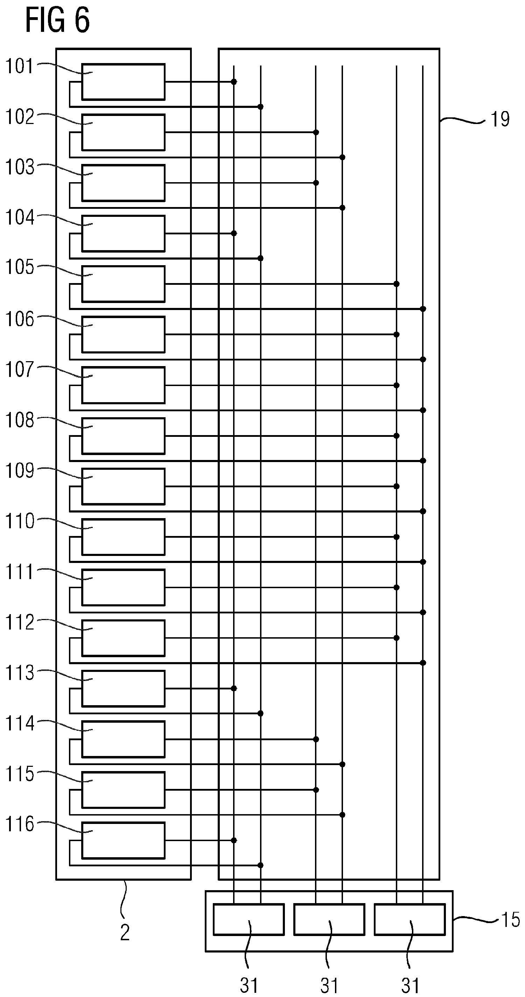

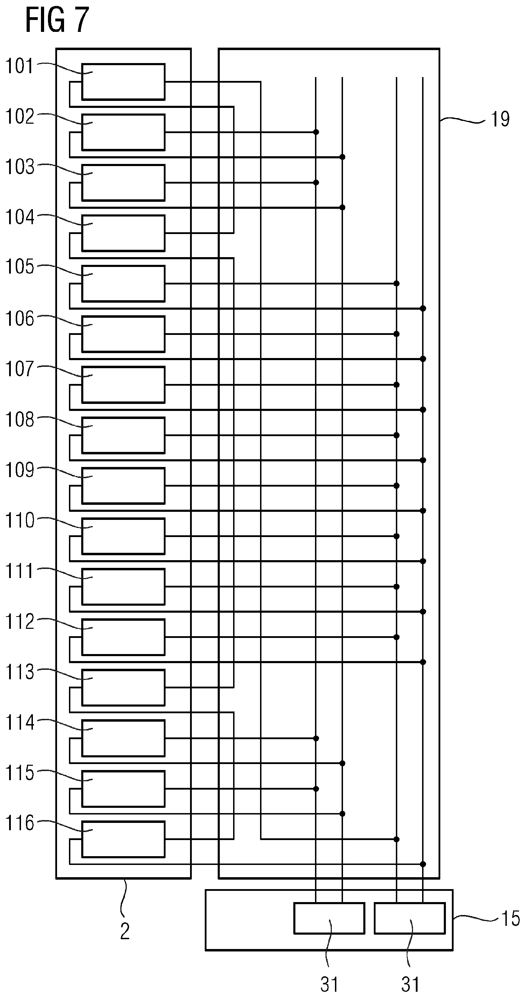

Fig. 6 und 7 zeigen exemplarische Verschaltungen der Teilheizbereiche 101,102,...116 einer Heizschicht 2 eines Detektormoduls 1 mittels der Verdrahtungseinheit 19, wenn diese wie in Fig. 2 dargestellt unterteilt ist. Die gezeigten exemplarischen Verdrahtungen der Teilheizbereiche 101,102,...116 berücksichtigen dabei eine angenommene Verteilung des Heizleistungsbedarfs in dem Stapelaufbau 5 wie anhand von Fig. 5 veranschaulicht. Dies ist jedoch rein exemplarisch. Es können in anderen Ausführungsvarianten auch andere Verdrahtungen abgestimmt auf die jeweils vorliegenden Bedingungen umgesetzt sein.

Fig. 6 zeigt mehrere Teilzahlen an Teilheizbereichen, welche mittels der Verdrahtungseinheit in Parallelschaltung jeweils an eine Leistungseinheit 31 auf einer Modulelektronik angeschlossen sind. Die erste Teilzahl umfasst die Teilheizbereiche 101, 104, 113 und 116. Die zweite Teilzahl umfasst die Teilheizbereiche 102, 103, 114 und 115. Die dritte Teilzahl umfasst die Teilheizbereiche 105 bis 112. Die Teilheizbereiche, welche jeweils gemeinsam an eine Leistungseinheit 31 angeschlossen sind, werden jeweils mit der gleichen Leistung versorgt. Durch eine derartige Verschaltung können jeweils alle Teilheizbereiche nach ihrem Heizleistungsbedarf mit Leistung versorgt werden, wobei kosteneffizient nur eine geringere Anzahl an Leistungseinheiten 31 notwendig sind.

Bei günstigen geometrischen Verhältnissen kann durch eine geeignete Kombination aus Reihen- und Parallelschaltung von Teilheizbereichen 101,102,...116 die Anzahl der notwendigen Leistungseinheiten 31 weiter reduziert werden. Mittels einer Verschaltung der zumindest einen Teilzahl der Teilheizbereiche 101,102,...116 mittels der Verdrahtungseinheit umfassend eine Kombination aus einer Parallelschaltung und einer Reihenschaltung, kann bei Versorgung mit Leistung durch eine gemeinsame Leistungseinheit 31 ein festes Verhältnis zwischen den in den jeweiligen Teilheizbereichen vorliegenden Teilleistungen eingestellt werden. Fig. 7 zeigt hierfür ein Beispiel, bei dem die Teilheizbereiche 101, 104, 113 und 116 sowie die Teilheizbereiche 105 bis 112 mittels einer Kombination aus Reihen- und Parallelschaltung an eine gemeinsame Leistungseinheit 31 angeschlossen sind. Dabei wird in den Teilheizbereichen 101, 104, 113 und 116 jeweils 1/16 der Leistung der anderen Teilheizbereiche 105 bis 112 an der gleichen Leistungseinheit 31 erreicht. Natürlich ist die Möglichkeit einer derartigen Verschaltung von der erwarteten Verteilung des Heizleistungsbedarfs und dessen zeitlichen Verlaufs abhängig, insofern, dass dadurch der Bruchteil an zugeführter Leistung in der Verdrahtung festgelegt ist und damit keine unabhängige, z.B. zeitlich variable, Anpassung innerhalb der miteinander derart verschalteten Teilzahl an Teilheizbereichen ermöglicht ist.

In vorteilhaften Ausbildungen eines erfindungsgemäßen Detektormoduls 1 wie anhand der Figuren zuvor beschrieben basiert die von einer jeweiligen Leistungseinheit 31 bereitgestellte Leistung für die an sie angeschlossenen Teilheizbereiche auf einer erwarteten und/oder gemessenen Temperatur im Stapelaufbau 5. Insbesondere kann vorteilhaft ein Temperaturverlauf während des Betriebs oder die Temperatur bei verschiedenen Betriebsparameter und/oder Umgebungsparameter des Detektormoduls 1 berücksichtigt werden. Insbesondere kann dies umfassen, dass eine Temperaturverteilung in einer zum Stapelaufbau 5 senkrechten Projektionsebene und ggf. auch deren zeitlicher Verlauf bzw. deren Abhängigkeit von verschiedenen Betriebs- und/oder Umgebungsparametern gemessen und/oder berechnet bzw. geschätzt wird, wobei die von einer jeweiligen Leistungseinheit bereitgestellte Leistung basierend auf der Temperaturverteilung eingestellt oder geregelt wird. Vorteilhaft kann die jeweils bereitgestellte Leistung einer Leistungseinheit 31 auf den vorliegenden Heizleistungsbedarf der dieser Leistungseinheit 31 zugeordneten Teilheizbereiche angepasst werden. Für eine Messung der Temperatur kann dabei insbesondere, wie auch in Fig. 2 angedeutet, mindestens ein Temperatursensor 30 im Stapelaufbau angeordnet sein. In vorteilhaften Ausbildungen sind mehrere Temperatursensoren 30 im Stapelaufbau angeordnet, so dass eine aktuelle Temperaturverteilung in einer zum Stapelaufbau senkrechten Projektionsebene anhand von Messwerten verbessert ortsaufgelöst erfasst werden kann. Darauf basierend kann eine Ansteuerung und/oder Regelung der in den Teilheizbereichen bereitgestellten Leistung umgesetzt sein. In einer vorteilhaft zweckmäßigen Ausführungsvariante ist der mindestens eine Temperatursensor 30 in die Ausleseschicht integriert.

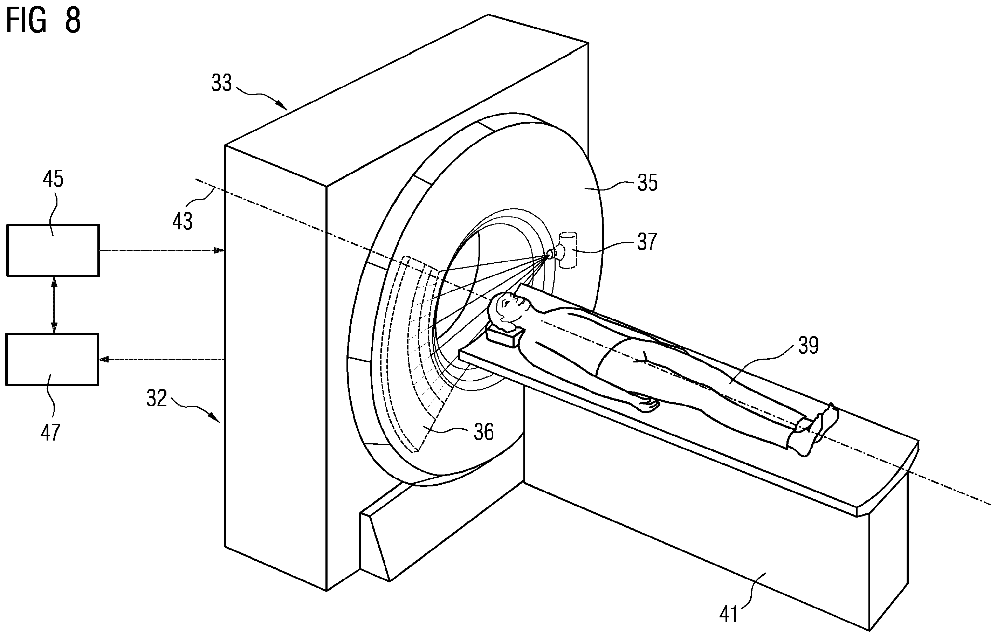

Fig. 8 zeigt eine beispielhafte Ausführungsform eines Computertomographiegeräts 32 mit einem Röntgendetektor 36 umfassend zumindest ein erfindungsgemäßes Detektormodul 1 und einer Röntgenquelle 37 in Gegenüberstellung dazu. Die Röntgenquelle 37 ist ausgebildet, den Röntgendetektor 36 mit Röntgenstrahlung zu belichten. Die Röntgenquelle 37 und der Röntgendetektor ist von einer Gantry 33 umfasst und auf einem Rotor 35 angeordnet. Der Rotor 35 ist um die Rotationsachse 43 drehbar. Das Untersuchungsobjekt 39, hier ein Patient, ist auf der Patientenliege 41 gelagert und ist entlang der Rotationsachse 43 durch die Gantry 33 bewegbar. Zur Steuerung des Computertomographiegeräts 32 und/oder zur Berechnung von Schnittbilder bzw. Volumenbildern des Objekts wird eine Recheneinheit 45 verwendet. Eine Rekonstruktionsvorrichtung 45 in Form eines Computersystems ist ausgebildet, Röntgenbilddaten basierend auf den Daten des Röntgendetektors 36 des Computertomographiegeräts zu rekonstruieren. Ein weiteres Computersystem dient als Bedienerkonsole 47. Die auf der Bedienerkonsole 47 installierte Software ermöglicht es dem Bediener, den Betrieb des Computertomographiegeräts zu steuern, wie z. B. die Auswahl eines Protokolls, das Starten des Scannens, usw. Die Rekonstruktionsvorrichtung 45 und die Bedienkonsole 47 können auch als ein Computersystem ausgebildet sein.

Der Röntgendetektor 36 eines solchen medizinischen Bildgebungsgerät 32 kann insbesondere ein Detektormodul 1 oder auch mehrere Detektormodule 1 umfassen. Insbesondere sind die Detektormodule 100 dann in der Regel zumindest in Rotationsrichtung nebeneinander angeordnet sind, so dass durch Aneinanderreihung der der jeweiligen Detektionsflächen der Detektormodule 100 insgesamt eine vorteilhaft große Gesamtdetektionsfläche ausgebildet werden kann.

## Patentansprüche

1. Detektormodul (1) für einen Röntgendetektor (36) umfassend in einem Stapelaufbau (5) eine Sensorschicht (11), eine Ausleseschicht (17), eine Heizschicht (2), sowie eine der Heizschicht (2) im Stapelaufbau direkt nachgelagerte, als von der Heizschicht (2) separates Bauteil ausgebildete Verdrahtungseinheit (19), wobei die Heizschicht (2) in eine Mehrzahl an Teilheizbereiche (101,102,...,116) unterteilt ist, welche jeweils zumindest ein Heizelement (27) aufweisen und jeweils einzeln für eine Versorgung mit Leistung kontaktierbar sind, wobei jeder Teilheizbereich (101,102,...,116) der Heizschicht (2) Kontaktstellen (29) zur Versorgung mit Leistung aufweist, und wobei mittels der nachgeordneten Verdrahtungseinheit (19) die Teilheizbereiche (101,102,...,116) der Heizschicht (2) jeweils kontaktiert sind, indem die Kontaktstellen (29) der Heizschicht (2) und jeweilige auf der Verdrahtungseinheit (19) vorgesehene Gegenkontaktstellen kontaktiert werden, und zumindest eine Teilzahl der Teilheizbereiche (101,102,...,116) für die Versorgung mit Leistung miteinander verschaltet sind.

2. Detektormodul (1) nach Anspruch 1, außerdem umfassend eine Anzahl an Leistungseinheiten (31), wobei die zumindest eine über die Verdrahtungseinheit (19) miteinander verschaltete Teilzahl an Teilheizbereichen (101,102,...,116) an eine gemeinsame Leistungseinheit (31) für die Versorgung mit Leistung angeschlossen ist.

3. Detektormodul (1) nach einem der vorangehenden Ansprüche, wobei die Verschaltung der zumindest einen Teilzahl an Teilheizbereichen (101,102,...,116) mittels der Verdrahtungseinheit (19) eine Parallelschaltung von zumindest zwei der Teilheizbereiche (101,102,...,116) oder eine Reihenschaltung von zumindest zwei der Teilheizbereiche (101,102,...,116) umfasst.

4. Detektormodul (1) nach einem der vorangehenden Ansprüche, wobei die Verschaltung der zumindest einen Teilzahl der Teilheizbereiche (101,102,...,116) mittels der Verdrahtungseinheit (19) eine Kombination aus einer Parallelschaltung und einer Reihenschaltung von Teilheizbereichen (101,102,...,116) der Teilzahl umfasst, so dass durch die Verschaltung bei Versorgung der Teilzahl der Teilheizbereiche (101,102,...,116) mit Leistung ein festes Verhältnis zwischen den in den jeweiligen Teilheizbereichen (101,102,...,116) vorliegenden Teilleistungen eingestellt ist.

5. Detektormodul (1) nach Anspruch 2, wobei die von einer jeweiligen Leistungseinheit (31) bereitgestellte Leistung für die an sie angeschlossenen Teilheizbereiche (101,102,...,116) basierend auf einer erwarteten und/oder gemessenen Temperatur im Stapelaufbau basiert.

6. Detektormodul (1) nach einem der vorangehenden Ansprüche, wobei im Stapelaufbau mindestens ein Temperatursensor (30) angeordnet ist.

7. Detektormodul (1) nach einem der vorangehenden Ansprüche, wobei die Heizschicht (2) auf einer Oberfläche der Ausleseschicht (17) aufgebracht oder in die Ausleseschicht (17) integriert ist.

8. Detektormodul (1) nach einem der vorangehenden Ansprüche, wobei die Verdrahtungseinheit (19) als Leiterplatte ausgebildet ist.

9. Detektormodul (1) nach einem der vorhergehenden Ansprüche, wobei die Sensorschicht (11) ein direkt konvertierendes Halbleitermaterial, insbesondere Cadmiumtellurid (CdTe) oder Cadmiumzinktellurid (CdZnTe), umfasst.

10. Detektormodul (1) nach einem der vorangehenden Ansprüche, wobei der Stapelaufbau (5) auf einem Modulträger (7) angeordnet ist, welcher über den Stapelaufbau (5) thermisch mit der Sensorschicht (11) gekoppelt ist.

11. Detektormodul (36) nach einem der vorhergehenden Ansprüche, wobei die Verdrahtungseinheit (19) im Stapelaufbau (5) über den Modulträger (7) mit einer Modulelektronik (15) verbunden ist.

12. Detektormodul (36) nach Anspruch 11 in Kombination mit Anspruch 2, wobei die Anzahl an Leistungseinheiten (31) von der Modulelektronik (15) umfasst sind.

13. Detektormodul (1) nach einem der Ansprüche 10 bis 12, mit einer Anzahl von einander benachbart auf dem Modulträger (7) angeordneten Stapelaufbauten (5).

14. Röntgendetektor (36) zur Bildaufnahme eines von Röntgenstrahlung durchstrahlten Objektes (39), umfassend eine Mehrzahl von einander benachbart angeordneten Detektormodulen (1) nach einem der vorangehenden Ansprüche.

15. Computertomographiegerät (32) umfassend einen Röntgendetektor (36) nach Anspruch 14 und einer Röntgenquelle (37) in Gegenüberstellung dazu, wobei die Röntgenquelle (37) ausgebildet ist, den Röntgendetektor (36) mit Röntgenstrahlung zu belichten.

## Claims

1. Detector module (1) for an X-ray detector (36) comprising in a stacked structure (5) a sensor layer (11), a readout layer (17), a heating layer (2), and a wiring unit (19) which is positioned directly after the heating layer (2) in the stacked structure and is embodied as a separate component from the heating layer (2), wherein the heating layer (2) is partitioned into a plurality of heating subregions (101,102,...,116), each of which comprises at least one heating element (27), and with each of which contact can be made individually for supplying power, wherein each heating subregion (101,102,...,116) of the heating layer (2) has contact points (29) for the supply of power, and wherein by means of the wiring unit (19) arranged after, contact is made to each of the heating subregions (101,102,...,116) of the heating layer (2), by contact being made between the contact points (29) of the heating layer (2) and respective mating contact points provided on the wiring unit (19), and at least one subset of the heating subregions (101,102,...,116) is interconnected for the supply of power.

2. Detector module (1) according to claim 1, also comprising a number of power units (31), wherein the at least one subset of the heating subregions (101,102,...,116) that is interconnected via the wiring unit (19) is connected to a shared power unit (31) for the supply of power.

3. Detector module (1) according to one of the preceding claims, wherein the interconnection of the at least one subset of the heating subregions (101,102,...,116) by means of the wiring unit (19) comprises a parallel connection of at least two of the heating subregions (101,102,...,116) or a series connection of at least two of the heating subregions (101,102,...,116).

4. Detector module (1) according to one of the preceding claims, wherein the interconnection of the at least one subset of the heating subregions (101,102,...,116) by means of the wiring unit (19) comprises a combination of a parallel connection and a series connection of heating subregions (101,102,...,116) of the subset, so that, when power is being supplied to the subset of the heating subregions (101,102,...,116), the interconnection sets a fixed ratio between the sub-powers present in the respective heating subregions (101,102,...,116).

5. Detector module (1) according to claim 2, wherein the power provided by a particular power unit (31) for the heating subregions (101,102,...,116) connected thereto is based on an expected and/or measured temperature in the stacked structure.

6. Detector module (1) according to one of the preceding claims, wherein at least one temperature sensor (30) is located in the stacked structure.

7. Detector module (1) according to one of the preceding claims, wherein the heating layer (2) is applied to a face of the readout layer (17) or integrated in the readout layer (17).

8. Detector module (1) according to one of the preceding claims, wherein the wiring unit (19) is embodied as a printed circuit board.

9. Detector module (1) according to one of the preceding claims, wherein the sensor layer (11) comprises a direct converting semiconductor material, in particular cadmium telluride (CdTe) or cadmium zinc telluride (CdZnTe).

10. Detector module (1) according to one of the preceding claims, wherein the stacked structure (5) is located on a module carrier (7), which is thermally coupled to the sensor layer (11) via the stacked structure (5).

11. Detector module (36) according to one of the preceding claims, wherein the wiring unit (19) in the stacked structure (5) is connected to a module electronic circuit (15) via the module carrier (7).

12. Detector module (36) according to claim 11 in combination with claim 2, wherein the number of power units (31) are comprised by the module electronic circuit (15).

13. Detector module (1) according to one of claims 10 to 12, having a number of stacked structures (5) located adjacent to one another on the module carrier (7).

14. X-ray detector (36) for acquiring images of an X-rayed object (39), comprising a plurality of adjacently located detector modules (1) according to one of the preceding claims.

15. Computed tomography device (32) comprising an X-ray detector (36) according to claim 14 and an X-ray source (37) opposite thereto, wherein the X-ray source (37) is designed to shine X-ray radiation onto the X-ray detector (36).

## Revendications

1. Module (1) de détecteur pour un détecteur (36) de rayons X comprenant suivant une structure (5) empilée une couche (11) de détecteur, une couche (17) de lecture, une couche (2) de chauffage, ainsi qu'une unité (19) de câblage montée directement en aval dans la structure empilée de la couche (2) de chauffage et constituée sous la forme d'un composant distinct de la couche (2) de chauffage, dans lequel la couche (2) de chauffage est subdivisée en une pluralité de zones (101, 102, ..., 116) partielles de chauffage, qui ont chacune au moins un élément (27) de chauffage et qui peuvent être mises en contact chacune individuellement pour une alimentation en puissance, dans lequel chaque zone (101, 102, ..., 116) partielle de chauffage de la couche (2) de chauffage a des points (29) de contact pour l'alimentation en puissance, et dans lequel, au moyen de l'unité (19) de câblage en aval, les zones (101, 102, ..., 116) partielles de chauffage de la couche (2) de chauffage sont contactées respectivement, par le fait que les points (29) de contact de la couche (2) de chauffage et des points de contact antagonistes prévus sur l'unité (19) de câblage sont mis en contact, et au moins un nombre partiel des zones (101, 102, ..., 116) partielles de chauffage sont connectées entre elles pour l'alimentation en puissance.

2. Module (1) de détecteur suivant la revendication 1, comprenant en outre un certain nombre d'unités (31) de puissance, dans lequel le au moins un nombre partiel de zones (101, 102, ..., 116) partielles de chauffage, connectées entre elles par l'intermédiaire de l'unité (19) de câblage, sont raccordées à une unité (31) de puissance commune pour l'alimentation en puissance.

3. Module (1) de détecteur suivant l'une des revendications précédentes, dans lequel la connexion du au moins un nombre partiel de zones (101, 102, ..., 116) partielles de chauffage au moyen de l'unité (19) de câblage comprend un montage en parallèle d'au moins deux des zones (101, 102, ..., 116) partielles de chauffage ou un montage en série d'au moins deux des zones (101, 102, ..., 116) partielles de chauffage.

4. Module (1) de détecteur suivant l'une des revendications précédentes, dans lequel la connexion du au moins un nombre partiel de zones (101, 102, ..., 116) partielles de chauffage au moyen de l'unité (19) de câblage comprend une combinaison d'un montage en parallèle et d'un montage en série de zones (101, 102, ..., 116) partielles de chauffage du nombre partiel, de sorte que, par la connexion d'une alimentation du nombre partiel de zones (101, 102, ..., 116) partielles de chauffage en puissance, il est établi un rapport fixe entre les puissances partielles présentes dans les zones (101, 102, ..., 116) partielles de chauffage respectives.

5. Module (1) de détecteur suivant la revendication 2, dans lequel la puissance, mise à disposition, par une unité (31) respective de puissance, des zones (101, 102, ..., 116) partielles de chauffage, qui y sont raccordées, repose sur une température escomptée et/ou mesurée dans la structure empilée.

6. Module (1) de détecteur suivant l'une des revendications précédentes, dans lequel au moins un capteur (30) de température est disposé dans la structure empilée.

7. Module (1) de détecteur suivant l'une des revendications précédentes, dans lequel la couche (2) de chauffage est déposée sur une surface de la couche (17) de lecture ou est intégrée à la couche (17) de lecture.

8. Module (1) de détecteur suivant l'une des revendications précédentes, dans lequel l'unité (19) de câblage est constituée sous la forme d'une plaquette à circuit imprimé.

9. Module (1) de détecteur suivant l'une des revendications précédentes, dans lequel la couche (11) de capteur comprend un matériau semiconducteur à conversion directe, en particulier du tellurure de cadmium (CdTe) ou du tellurure de cadmium et de zinc (CdZnTe).

10. Module (1) de détecteur suivant l'une des revendications précédentes, dans lequel la structure (5) empilée est disposée sur un support (7) de module, par lequel la structure (5) empilée est couplée thermiquement à la couche (11) de capteur.

11. Module (36) de détecteur suivant l'une des revendications précédentes, dans lequel l'unité (19) de câblage est reliée dans la structure (5) empilée à une électronique (15) de module par l'intermédiaire du support (7) de module.

12. Module (36) de détecteur suivant la revendication 11 en combinaison avec la revendication 2, dans lequel le nombre d'unités (31) de puissance sont comprises par l'électronique (15) de module.

13. Module (1) de détecteur suivant l'une des revendications 10 à 12, comprenant un nombre de superstructures (5) de pile disposées au voisinage les unes des autres sur le support (7) de module.

14. Détecteur (36) de rayons X pour l'enregistrement d'image d'un objet (39) traversé par du rayonnement X, comprenant une pluralité de modules (1) de détecteur disposés au voisinage les uns des autres suivant l'une des revendications précédentes.

15. Appareil (32) de tomodensitométrie assisté par ordinateur, comprenant un détecteur (36) de rayons X suivant la revendication 14 et une source (37) de rayons X en regard de celui-ci, dans lequel la source (37) de rayons X est constituée pour éclairer le détecteur (36) de rayons X par du rayonnement X.
